# EUROPÄISCHE PATENTSCHRIFT

(11) **EP 2 125 209 B1**
(45) Veröffentlichungstag und Bekanntmachung des Hinweises auf die Patenterteilung: **06.09.2017**
(21) Anmeldenummer: 08734665.6
(22) Anmeldetag: 19.03.2008
(51) Int. Cl.: C07C 51/41, B01J 31/04, B01J 31/22, C07F 15/00

(54) **KATALYTISCH HOCHWIRKSAME EDELMETALL-CARBOXYLAT-VERBINDUNGEN VON RU UND IR**
CATALYTICALLY HIGHLY EFFECTIVE PRECIOUS METAL-CARBOXYLATE COMPOUNDS OF RU AND IR
COMPOSÉS MÉTAL NOBLE-CARBOXYLATE DE RU ET IR À HAUTE EFFICACITÉ CATALYTIQUE

(30) Priorität: 26.03.2007 DE 102007014914
(43) Veröffentlichungstag der Anmeldung: 02.12.2009
(73) Patentinhaber: Heraeus Deutschland GmbH & Co. KG, 63450 Hanau (DE)
(72) Erfinder: WALTER, Richard, 63755 Alzenau (DE); MEYER, Horst, 63674 Altenstadt (DE); VOSS, Steffen, 63694 Limeshain (DE); SCHAPP, Jan, 63303 Dreieich (DE)
(74) Vertreter: Gille Hrabal
(86) Internationale Anmeldenummer: PCT/EP2008/002186
(87) Internationale Veröffentlichungsnummer: WO 2008/116585

(56) Entgegenhaltungen:
- EP-A- 0 844 251
- EP-A- 1 046 629
- WO-A-96/23757
- WO-A-2006/125628
- GB-A- 2 413 323

## Beschreibung

Die Erfindung betrifft die Verwendung einer Edelmetall-Carboxylat-Verbindung von Ru oder Ir oder einer Lösung dessen als Katalysator.

Eine bekannte Verwendung von Iridium, Ruthenium und deren Verbindungen ist die als Katalysator und Katalysatorvorläufer. So werden z.B. nach EP 0 616 997 A1 und EP 0 786 447 A1 Iridium-Katalaysatoren bei Carbonylierungsreaktionen eingesetzt. Als für diesen Zweck geeignete Iridiumverbindung wird u. a. Iridiumacetat genannt.

Aus der deutschen Auslegeschrift 1 127 888 ist bekannt, Vinylester höherer Carbonsäuren durch Umesterung der Vinylester niederer Carbonsäuren in Gegenwart von Platingruppenmetallsalzen herzustellen. Beispiele für geeignete Platingruppenmetallsalze sind u. a. die Acetate des Palladiums und des Rhodiums, die aus den in Eisessig gelösten Hydroxiden durch Umsetzung mit Essigsäureanhydrid erhalten werden.

EP 0 844 251 betrifft die Herstellung von Ruthenium-Carboxylat-Lösungen aus Ruthenium (IV) Oxid unter Reduktion mit Hydrazinderivat in Gegenwart einer Carbonsäure. Die Herstellung von Rutheniumoxid, insbesondere dessen Filtration ist sehr aufwändig.

In EP 1 046 629 wird ein Verfahren zur Herstellung von Ir-Acetat beschrieben, wobei aus einer wässrigen Lösung einer Iridiumchloroverbindung mit einer wässrigen Lösung eines Alkalimetallhydroxids, -carbonats oder -hydrogencarbonats Iridiumhydroxid gefällt, das gefällte Iridiumhydroxid abgetrennt und mit Essigsäure oder einem Gemisch aus Essigsäure/Essigsäureanhydrid-Gemisch zu einer Iridiumacetat enthaltenen Lösung umgesetzt und das Iridiumacetat als Feststoff aus der Lösung isoliert wird.

Watson beschreibt die Herstellung von Ir-Acetat (Precious Metals Catalysts Seminar, S. 83 ff.), wobei Ir-Schwamm mit Bariumperoxid gesintert wird. Anschließend wird die Sintermasse mit einem Gemisch aus Essigsäure/Wasser gelöst und von nicht abreagiertem Metall durch Filtration getrennt. Dann wird Bariumsulfat durch Zusatz von Schwefelsäure ausgefällt und durch Filtration von der Iridiumacetat-Lösung getrennt. Darauf wird die Iridiumacetat-Lösung durch Eindampfen aufkonzentriert. Ein Schlüsselschritt dieses Prozesses ist die vorsichtige Balance bei der Entfernung des Bariums. Weder Barium noch Sulfate sind im Endprodukt erwünscht. Daher ist eine sehr genaue Einstellung erforderlich, um das gewünschte Produkt zu erhalten.

Dieses Verfahren hat mehrere Nachteile:
Die genaue Einstellung des Schwefelsäure-Gehalts ist schwierig und muss analytisch geprüft werden. Daher sind in der Regel mehrmalige Fällungen und Filtrationen von Ba-Sulfat mit den entsprechenden analytischen Untersuchungen nötig. Ein weiterer Nachteil ist die äußerst schwierige und kostenintensive Filtration des Ba-Sulfats. Zudem lässt sich das enthaltene Strontium über diesen Fällungsweg nicht hinreichend abreichern und begrenzt daher die Produktreinheit.

Es ist die Aufgabe der Erfindung katalytisch hochwirksame Edelmetall-Carboxylat-Verbindungen von Iridium und Ruthenium als Katalysator bereitzustellen. Die Edelmetall-Carboxylat-Verbindungen, insbesondere die Acetat-Verbindungen, sollen katalytisch möglichst hochwirksam sein. Vorzugsweise sollen die Verbindungen auch hohe Reinheit aufweisen, insbesondere arm an Chlor, Schwefel, Natrium und Kalium sein.

Gelöst wird diese Aufgabe durch die Verwendung einer Edelmetall-Carboxylat-Verbindung von Ru oder Ir oder einer Lösung dessen als Katalysator, wobei die Edelmetall-Carboxylat-Verbindung oder dessen Lösung mittels eines Verfahrens hergestellt worden ist, bei dem das Edelmetall mit Bariumperoxid aufgeschlossen wird und die Aufschlussmasse in einer Carbonsäure oder einer mit einem protischen Lösungsmittel verdünnten Carbonsäure gelöst wird, wobei aus der hieraus resultierenden Lösung die Erdalkaliionen als Salz der Oxalsäure abgetrennt werden, und wobei das Verfahren keine Bariumsulfat-Fällung und Filtration von Bariumsulfat umfasst. Dieses Verfahren ist sehr effizient und umgeht die aufwendige BaSO₄-Fällung und Filtration von Bariumsulfat nach dem Watson-Verfahren. Die gereinigte Edelmetall-Carboxylatlösung kann aufkonzentriert werden, wobei die Aufkonzentrierung auf eine gewünschte Konzentration eingestellt werden kann oder bis zur Isolierung von Feststoff erfolgen kann.

Dazu werden die dem Stand der Technik entsprechenden Methoden wie Eindampfen der Reaktionslösung, Sprühtrocknen oder Fällung bei tiefer Temperatur oder Zusatz eines geeigneten Solvens eingesetzt. Überraschenderweise sind die katalytischen Eigenschaften besonders gut, wenn eine Edelmetall-Carboxylat-Lösung, insbesondere eine Essigsäure-Lösung von Rutheniumacetat oder Iridiumacetat aus dem Verfahren mit der Fällung des Erdalkalimetalls als Salz der Oxalsäure stammt. In einer so hergestellten Lösung ist die Masse an Oxalsäure, bzw. Salzen der Oxalsäure kleiner oder gleich der Masse an Edelmetall. Die Edelmetalle werden mit Bariumperoxid aufgeschlossen. Edelmetalle sind Ruthenium und Iridium. Das bevorzugte Carboxylat ist das Acetat. Entsprechend bevorzugt wird das aufgeschlossene Edelmetallgemisch in wässriger Essigsäure gelöst. Erfindungsgemäß beträgt der Anteil an Erdalkalimetall, insbesondere Calcium, Strontium oder Barium nach der Fällung als Salz der Oxalsäure 100 ppm bis 10 Gew.-%, insbesondere 500 ppm bis 0,5 Gew.-%, bezogen auf das Edelmetall. Der Calcium, Strontium und Bariumgehalt kann durch fraktionierte Kristallisation maßgeblich reduziert werden.

### Beispiel 1:

1,5 g Ruthenium-Pulver (14,8 mmol) und 10 g Bariumperoxid (Reinheit 95,8 %; 56,5 mmol) werden gemischt.

Das Gemisch wird in einen Nickel-Tiegel überführt und bei 850°C 15 Stunden lang erhitzt. In einem 200 ml-Dreihalskolben werden 150 ml eines Gemisches aus Essigsäure und Wasser im Verhältnis 2:1 auf 50°C erhitzt.

Das Reaktionsgemisch aus Ruthenium-Pulver und Bariumperoxid wird in den Dreihalskolben eingetragen. Durch die exotherme Reaktion erhitzt sich das Gemisch auf 80°C. Die Mischung wird auf Siedetemperatur erhitzt und 3 Stunden bei Siedetemperatur gerührt.

Anschließend wird die Lösung auf Raumtemperatur abgekühlt und über Membranfilter filtriert. Der Filterkuchen wird mit wenig Essigsäure/Wasser-Gemisch (2:1) gewaschen. Man erhält 169 g Lösung mit einem Ru-Gehalt von 0,88 % (1,487 g Ru, 14,7 mmol) und einem Ba-Gehalt von 4,59 % (7,757 g Ba, 56,5 mmol).

169 g Ru-Acetat-Lösung (1,487 g Ru, 14,7 mmol) wird in einem 200 ml-Dreihalskolben überführt und unter Rühren mit 14,264 g Oxalsäuredihydrat (113 mmol) versetzt. Nach 4 Stunden Rührzeit wird die Suspension über Schwarzbandfilter und anschließend über Membranfilter filtriert. Der Filterkuchen wird mit Essigsäure/Wasser-Gemisch (2:1) gewaschen.

Man erhält 230 g Lösung mit einem Ru-Gehalt von 0,57 % (1,311 g Ru, 13 mmol) und einem Ba-Gehalt von 0,051 % (0,117 g, 0,85 mmol). Der Gehalt an Oxalat (Gehaltsbestimmung mittels lonenchromatographie) beträgt 0,36 %.

### Beispiel 2

1,5 g Ruthenium-Pulver (14,8 mmol) und 10 g Bariumperoxid (Reinheit 95,8 %; 56,5 mmol) werden gemischt.

Das Gemisch wird in einen Nickel-Tiegel überführt und bei 850°C 15 Stunden lang erhitzt. In einem 200 ml Dreihalskolben werden 150 ml eines Gemisches aus Essigsäure und Wasser im Verhältnis 2:1 auf 50°C erhitzt.

Das Reaktionsgemisch aus Ruthenium-Pulver und Bariumperoxid wird in den Dreihalskolben eingetragen. Durch die exotherme Reaktion erhitzt sich das Gemisch auf 76°C. Die Mischung wird auf Siedetemperatur erhitzt und 3 Stunden bei Siedetemperatur gerührt. Anschließend wird die Lösung auf Raumtemperatur abgekühlt und über Membranfilter filtriert. Der Filterkuchen wird mit wenig Essigsäure gewaschen.

Man erhält 166 g Lösung mit einem Ru-Gehalt von 0,90 % (1,494 g Ru, 14,8 mmol) und einem Ba-Gehalt von 4,67 % (7,752 g Ba, 56,4 mmol).

169 g Ru-Acetat-Lösung (1,494 g Ru, 14,8 mmol) wird in einem 200 ml Dreihalskolben überführt und unter Rühren mit 14,264 g Oxalsäuredihydrat (113 mmol) versetzt. Nach 4 Stunden Rührzeit wird die Suspension über Schwarzbandfilter und anschließend über Membranfilter filtriert. Der Filterkuchen wird mit Essigsäure gewaschen.

Man erhält 193 g Lösung mit einem Ru-Gehalt von 0,68 % (1,314 g Ru, 13 mmol) und einem Ba-Gehalt von 0,0515 % (0,1 g, 0,72 mmol). Der Gehalt an Oxalat (Gehaltsbestimmung mittels lonenchromatographie) beträgt 0,26 %.

### Beispiel 3(nicht erfindungsgemäß)

5,001 g Rhodiumpulver und 34,622 g Bariumperoxid wurden in einem Schraubdeckelglas eingewogen und 10 Minuten gut durchgemischt. Das Gemisch wurde in einen Nickeltiegel überführt und darauf in einem Muffelofen 15 Stunden bei 850°C erhitzt. Danach wurde der Tiegel auf Raumtemperatur abgekühlt. Die Schmelze ist an ihrer Oberfläche schwarz, darunter homogen und grau. Die Schmelze wurde in eine Lösung aus Essigsäure und vollentsalztem Wasser gegeben und gerührt, wobei das Verhältnis von Essigsäure zu Wasser 2 : 1 Volumenanteile beträgt. Dabei stieg die Temperatur auf 24°C an. Man erhielt eine dunkelgrüne Flüssigkeit mit schwarzen Partikeln. Nach 15minütigem weiteren Rühren wurde die Mischung auf 58°C erhitzt und 5 Stunden bei dieser Temperatur gehalten. Dabei nahm die Suspension innerhalb von ungefähr 5 Minuten eine braune Farbe an. Danach wurde mit 51,453 Gramm Oxalsäuredihydrat das Barium als Salz gefällt und eine Stunde nachgerührt, worauf eine gelbe Flüssigkeit über eine Nutsche mit Blaubandfilter abfiltriert wurde. Das Filtrat wurde am Rotationsverdampfer bei einer Temperatur von 75°C eingeengt. Das Rhodiumacetat wurde bis zur Massenkonstanz getrocknet und mit einem Mörser gemahlen. Die getrocknete Ausbeute an Rhodiumacetat betrug 11,012 g. Der Rhodiumgehalt wurde mittels ICP-Analyse auf 34,43 Gew.-% ermittelt.

### Beispiel 4

Iridium wird gemäß dem Watson-Prozess mit Bariumperoxid aufgeschlossen und mit Essigsäure gelöst. Das Barium aus dem Watson-Prozess wird analog zu den vorstehenden Beispielen 1 bis 3 mit Oxalsäure ausgefällt und abfiltriert. Derartige Iridium-Carboxyl-Verbindungen, insbesondere Acetate, weisen unübertroffene katalytische Eigenschaften auf.

## Patentansprüche

1. Verwendung einer Edelmetall-Carboxylat-Verbindung von Ru oder Ir oder einer Lösung dessen als Katalysator, wobei die Edelmetall-Carboxylat-Verbindung oder dessen Lösung mittels eines Verfahrens hergestellt worden ist, bei dem das Edelmetall mit Bariumperoxid aufgeschlossen wird und die Aufschlussmasse in einer Carbonsäure oder einer mit einem protischen Lösungsmittel verdünnten Carbonsäure gelöst wird, wobei aus der hieraus resultierenden Lösung die Erdalkaliionen als Salz der Oxalsäure abgetrennt werden, und wobei das Verfahren keine BaSO₄-Fällung und Filtration von Bariumsulfat umfasst.

2. Verwendung nach Anspruch 1, **dadurch gekennzeichnet, dass** die Carbonsäure Essigsäure ist.

3. Verwendung nach Anspruch 1 oder 2, **dadurch gekennzeichnet, dass** das Edelmetall-Carboxylat ein Edelmetall-Acetat ist.

4. Verwendung nach einem der vorhergehenden Anspruche, **dadurch gekennzeichnet, dass** die Edelmetall-Carboxylat-Verbindung die Form eines Stoffs hat, umfassend
A) das Edelmetall-Carboxylat,
B) mindestens eine Verbindung, ausgewählt aus der Gruppe bestehend aus Oxalsäure und einem Salz der Oxalsäure, und
C) Bariumionen.

5. Verwendung nach Anspruch 4, **dadurch gekennzeichnet, dass** der Stoff Essigsäure aufweist.

6. Verwendung nach Anspruch 4 oder 5, **dadurch gekennzeichnet, dass** der Stoff Wasser oder einen Alkohol aufweist.

7. Verwendung nach einem der Ansprüche 4 bis 6, **dadurch gekennzeichnet, dass** der Stoff eine Essigsäurelösung ist.

## Claims

1. A use of a precious metal carboxylate compound of Ru or Ir or a solution thereof as a catalyst, wherein the precious metal carboxylate compound or the solution thereof has been produced by means of a method, in which the precious metal is digested with barium peroxide and the digestion mass is dissolved in a carboxylic acid or a carboxylic acid diluted with a protic solvent, wherein the alkaline earth ions are separated from the solution resulting therefrom as salts of the oxalic acid, and wherein the method does not include any BaSO₄ precipitation and filtration of barium sulphate.

2. A use according to claim 1, **characterized in that** the carboxylic acid is acetic acid.

3. A use according to claim 1 or 2, **characterized in that** the precious metal carboxylate is a precious metal acetate.

4. A use according to one of the preceding claims, **characterized in that** the precious metal carboxylate compound has the form of a substance, including
A) the precious metal carboxylate
B) at least one compound selected from the group comprising oxalic acid and a salt of the oxalic acid, and
C) barium ions.

5. A use according to claim 4, **characterized in that** the substance comprises acetic acid.

6. A use according to claim 4 or 5, **characterized in that** the substance comprises water or an alcohol.

7. A use according to one of the claims 4 through 6, **characterized in that** the substance is an acetic acid solution.

## Revendications

1. Utilisation d'un composé de carboxylate de métal précieux de Ru ou de Ir ou d'une solution de celui-ci comme catalyseur, le composé de carboxylate de métal précieux ou la solution de celui-ci ayant été produit par moyen d'un procédé, dans lequel le métal précieux est digéré à l'aide de peroxyde de baryum et la masse de digestion est dissoute dans un acide carboxylique ou dans un acide carboxylique diluée avec un solvant protique, les ions alcalino-terreux étant séparés de la solution résultant de cela comme des sels de l'acide oxalique, et le procédé ne comprenant pas de précipitation de BaSO₄ et pas de filtration de sulfate de baryum.

2. Utilisation selon la revendication 1, **caractérisé en ce que** l'acide carboxylique est de l'acide acétique.

3. Utilisation selon la revendication 1 ou la revendication 2, **caractérisé en ce que** le carboxylate de métal précieux est un acétate de métal précieux.

4. Utilisation selon l'une des revendications précédentes, **caractérisé en ce que** le composé de carboxylate de métal précieux a la forme d'une substance, comprenant
A) le carboxylate de métal précieux,
B) au moins un composé sélectionné dans le groupe composé d'acide oxalique et d'un sel de l'acide oxalique, et
C) des ions de baryum.

5. Utilisation selon la revendication 4, **caractérisé en ce que** la substance comprend de l'acide acétique.

6. Utilisation selon la revendication 4 ou la revendication 5, **caractérisé en ce que** la substance comprend de l'eau ou un alcool.

7. Utilisation selon l'une des revendications 4 à 6, **caractérisé en ce que** la substance est une solution d'acide acétique.
